(19)

Europäisches
Patentamt
European
Patent Office
Office européen
des brevets

(11) **EP 4 088 728 A1**

(12) **EUROPEAN PATENT APPLICATION**
published in accordance with Art. 153(4) EPC

(43) Date of publication:
**16.11.2022 Bulletin 2022/46**

(21) Application number: **21738178.9**

(22) Date of filing: **08.01.2021**

(51) International Patent Classification (IPC):
**A61K 35/28** (2015.01)          **A61K 9/00** (2006.01)
**A61P 25/28** (2006.01)

(52) Cooperative Patent Classification (CPC):
**A61K 9/00; A61K 35/28; A61P 25/28**

(86) International application number:
**PCT/KR2021/000263**

(87) International publication number:
**WO 2021/141446 (15.07.2021 Gazette 2021/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **08.01.2020  KR 20200002758**

(71) Applicant: **Corestem Co., Ltd.
Seongnam-si, Gyeonggi-do 13486 (KR)**

(72) Inventors:
• **KIM, Kyung Suk
Seoul 02838 (KR)**
• **LEE, Tae Yong
Yongin-si, Gyeonggi-do 17128 (KR)**
• **SUK, Kyoung Ho
Daegu 42040 (KR)**
• **LEE, Ho Won
Daegu 42119 (KR)**
• **KIM, Sang Ryong
Daegu 41560 (KR)**

(74) Representative: **HGF
HGF Limited
1 City Walk
Leeds LS11 9DX (GB)**

(54) **COMPOSITION FOR TREATING NEURODEGENERATIVE DISORDERS COMPRISING MESENCHYMAL STEM CELLS**

(57)    The present invention relates to a method or a composition for treating neurodegenerative diseases, and particularly, the present invention relates to a method for treating neurodegenerative diseases of the cerebellum, comprising administering a composition comprising stem cells as an active ingredient to a patient having a neurodegenerative disease of the cerebellum, or to a composition for treating neurodegenerative diseases of the cerebellum comprising mesenchymal stem cells as an active ingredient. The treatment method or the treatment composition using mesenchymal stem cells, according to the present invention, has remarkable effects in reducing neuroinflammation, inhibiting M1 microglia, activating M2 microglia, inhibiting apoptosis of Purkinje cells, inhibiting death of neurons, improving motor ability, and the like, and therefore may be effectively utilized in alleviating and treating neurodegenerative diseases including cerebellar ataxia and multiple system atrophy.

FIG. 5A

EP 4 088 728 A1

## Description

## Technical Field

This application claims priority to Korean Patent Application No. 10-2020-0002758 filed on January 8, 2020, which is incorporated herein by reference in its entirety.

The present invention relates to a method or a composition for treating neurodegenerative diseases, and particularly, the present invention relates to a method for treating neurodegenerative diseases of the cerebellum, comprising administering a composition comprising mesenchymal stem cells as an active ingredient to a patient having a neurodegenerative disease of the cerebellum, or to a composition for treating neurodegenerative diseases of the cerebellum comprising mesenchymal stem cells as an active ingredient.

## Background Art

Diseases such as cerebellar ataxia (CA) and multiple system atrophy (MSA) are motor neurodegenerative diseases caused by pathological processes affecting the cerebellum or related pathways. Cerebellar ataxia and multiple system atrophy are characterized by abnormal coordination and imbalance of movements, such as gait disturbance.

Cerebellar ataxia and multiple system atrophy can be caused by genetic defects, sporadic neurodegeneration disorders, acquired diseases (for example, infection, toxic reaction, alcohol, and vitamin deficiency), and other unknown causes. A number of recent clinical and preclinical studies have demonstrated that cerebellar inflammation may play an important role in the progression of cerebellar ataxia and multiple system atrophy. For example, neurotoxic inflammatory responses involving the production of inflammatory molecules by activation of microglia and astrocytes have been observed in patients and animal models of hereditary cerebellar ataxia, particularly spinocerebellar ataxia. Activation of neuroglia leads to the production of inflammatory molecules, and has been observed in many animal models of cerebellar ataxia and multiple system atrophy, as well as activation of neuroglia was found in the cerebellum of patients having cerebellar ataxia and multiple system atrophy. Cerebellar inflammation is known to induce the loss of Purkinje cells, which results in cerebellar dysfunction. In addition, the cerebellum is particularly lethal to poisons and poisoning, and acquired cerebellar ataxia and multiple system atrophy may occur due to inflammatory responses caused by viral infection and poison exposure (alcohol, drug, and environmental toxicity). However, although some animal models of the disease have been developed from the results of studies on the pathogenesis of cerebellar ataxia and multiple system atrophy related to these genetic causes, cerebellar neurotoxicity, and physical damage, there is no suitable animal model for studying cerebellar ataxia and multiple system atrophy in vivo.

The fundamental mechanism of neurodegenerative diseases including cerebellar ataxia and multiple system atrophy is the gradual appearance of functional and quantitative damage to nerve cells. Currently, neurodegenerative diseases such as cerebellar ataxia and multiple system atrophy cannot be prevented or treated, and only in extremely exceptional cases, treatment or symptom relief is possible.

Recently, mesenchymal stem cells (MSCs) are attracting attention as therapeutic candidates for neurodegenerative diseases because of their anti-inflammatory function, regenerative ability, and low immunoreactivity. Human mesenchymal stem cells (hMSCs) are multipotent cells having the ability to self-regenerate and differentiate into several specialized cells. In the damaged brain, hMSCs selectively target the site of damage and secrete various growth factors, cytokines, and chemokines known to have a wide range of activities, such as anti-apoptosis, angiogenesis, anti-inflammation, immunomodulation, and chemoattraction. Although hMSCs are known to have therapeutic effects on neurological diseases such as Alzheimer's disease, Parkinson's disease, and stroke, there is little information on the therapeutic effect of mesenchymal stem cells in patients and animal models of a neurodegenerative disease of the cerebellum.

Accordingly, the present inventors established an animal model by directly injecting lipopolyssacharide (LPS) into the cerebellum or by injecting Ara-C (cytarabine) into the abdominal cavity in order to confirm the effect of hMSCs in neurodegenerative diseases of the cerebellum including cerebellar ataxia and multiple system atrophy. In addition, the present inventors tried to confirm the therapeutic effect of human mesenchymal stem cells (hMSCs) in the three animal models including the SCA2 disease animal model, which is a genetic animal model through genetic modification.

## Detailed Description of Invention

## Technical Problem

An object of the present invention is to provide a method for treating neurodegenerative diseases of the cerebellum, comprising administering a composition comprising stem cells as an active ingredient to a patient having a neurodegenerative disease of the cerebellum.

Another object of the present invention is to provide a composition for alleviating or treating symptoms of neurodegenerative diseases of the cerebellum, comprising stem cells as an active ingredient.

However, the technical problem to be achieved by the present invention is not limited to the above-mentioned problems, and other problems not mentioned will be clearly understood by one of ordinary skill in the art from the following description.

**Solution to Problem**

In order to solve the above-mentioned problems, the present invention provides a method for treating neurodegenerative diseases of the cerebellum, comprising administering a composition comprising stem cells as an active ingredient to a patient having a neurodegenerative disease of the cerebellum, or a composition for alleviating or treating symptoms of neurodegenerative diseases of the cerebellum, comprising stem cells as an active ingredient.

The present invention established an animal model of a neurodegenerative disease by administering LPS to the animal model to induce inflammation in the cerebellum, or by administering Ara-C to inhibit the development of the cerebellum, and constructed an animal model with SCA2 genetic modification disease that is genetically stabilized through the fifth generation. Next, the therapeutic effect of mesenchymal stem cells (MSCs) was experimented in three animal models of a neurodegenerative disease having these various pathological mechanisms.

As used herein, the term "neurodegenerative disease of the cerebellum" refers to a neurological disease in which motion is clumsy and there is no coordination between motions due to abnormal functions of the cerebellum, and includes all neurodegenerative diseases induced by various medical and neurological diseases or genetic predisposition. In the present invention, the neurodegenerative disease of the cerebellum includes a neurodegenerative disease induced by inflammation, a neurodegenerative disease induced by toxins, or a neurodegenerative disease caused by genetic modification.

In the present invention, the neurodegenerative disease of the cerebellum includes cerebellar ataxia or multiple system atrophy.

As used herein, the term "stem cell" refers to an undifferentiation cell having the ability to differentiate into various body tissues, and more specifically, may include an undifferentiated cell, which has the ability to differentiate into any specific or a plurality of functional cells and the self-replicating ability to repeatedly produce the same cells themselves. It is generated in all tissues during the development of a fetus, and may be found in some tissues where cells are actively replaced, such as bone marrow and epithelial tissue, even in adulthood.

The stem cell of the present invention may be an adult stem cell, and the adult stem cell may be at least one selected from the group consisting of a mesenchymal stem cell (MSC), a mesenchymal stromal cell, and a multipotent stem cell, but is not limited thereto.

The stem cell of the present invention may be a human mesenchymal stem cell (hMSC), but is not limited thereto.

In addition, as used herein, the term "mesenchymal stem cell (MSC)" refers to a stem cell (multipotent stem cell) that has multipotency before being differentiated into a cell of a specific organ such as bone, cartilage, fat, nerve tissue, fibroblast, and a muscle cell. The mesenchymal stem cell may be preferably derived from one selected from the group consisting of umbilical cord, umbilical cord blood, bone marrow, fat, muscle, nerve, skin, amnionic membrane, chorion, decidua, and placenta, but is not limited thereto.

In one embodiment of the present invention, as the stem cell, an allogeneic bone marrow-derived mesenchymal stem cell was used.

In order to obtain the stem cells of the present invention, bone marrow, which is donated from normal and healthy donors by a method of collecting and isolating bone marrow, is diluted 1:2 with CSBM-A06 medium and then instilled on the Ficoll layer of the same amount of bone marrow prepared in advance. It is centrifuged at $400 \times$ g for 30 minutes, and the obtained monocyte cell layer may be isolated, washed twice with CSBM-A06 medium, and then cultured under culture conditions in which 37°C and 5% $CO_2$ is maintained. After 48 hours of culture, cells not attached to the bottom of the flask are removed by exchanging a new medium, and cells attached to the bottom are cultured by changing the medium once every 3 to 4 days. When the cultured cells grew to about 80%, they were subcultured into a new flask, and some of the cultured cells were continuously subcultured, and some were suspended in a cryopreservation solution containing DMSO and stored frozen.

In the present invention, as a culture medium usable for isolation and culture of stem cells, a cell culture medium containing 10% FBS may be used. As a cell culture medium, any cell culture medium commonly used in the art, such as Dulbecco's modified Eagle medium (DMEM), minimal essential medium (MEM), alpha-minimal essential medium (a-MEM), McCoys 5A medium, Eagle's basal medium, CMRL (Connaught Medical Research Laboratory) medium, Glasgow's minimal essential medium, Ham's F-12 medium, IMDM (Iscove's modified Dulbecco's medium), Liebovitz' L-15 medium, and RPMI (Roswell Park Memorial Institute) 1640 medium, may be used.

80% or more, preferably 90% or more of the stem cells according to the present invention express a mesenchymal stem cell-positive surface marker including CD29, CD44, CD73, CD90 or CD105, and 5% or less, preferably 3% or less of the stem cells according to the present invention express a negative surface marker including CD34 or CD45.

The stem cells according to the present invention, when directly transplanted into the cisterna magna of an animal model with a neurodegenerative disease, may induce anti-inflammatory activity of the cerebellum and reduce the expression of inflammatory cytokines (IL-1β or TNFα) or inflammatory chemokines (MIP-1α or MCP-1). In addition, the stem cells according to the present invention may inhibit the activation of M1-type microglia, which are inflammatory neuroglia, and activate M2-type microglia, which are anti-inflammatory neuroglia, thereby inducing anti-inflammatory activity of the cerebellum. In addition, the stem cells according to the present invention inhibit damage of Purkinje cell, and thus may be usefully used to alleviate or treat abnormal behavioral motor disorder symptoms. The composition of the present invention may further contain one or more known active ingredients having a therapeutic effect of neurodegenerative diseases such as multiple system atrophy together with the stem cells.

The composition of the present invention may include an appropriate suspending agent commonly used in the preparation of therapeutic compositions. For example, an injection may further include a preservative, an analgesic agent, a solubilizer or a stabilizer, etc., and a formulation for topical administration may further include a base, an excipient, a lubricant, or a preservative, etc.

As used herein, the term "administration" means providing a given composition of the present invention to a subject by any appropriate method. For a desirable effect, the dosage of stem cells is $2.0 \times 10^4$ to $1.0 \times 10^6$ cells/animal, and may be administered once or divided into several times for single administration or repeated administration. However, it should be understood that the actual dosage of the active ingredient should be determined in view of several related factors such as the disease to be treated, the severity of the disease, the route of administration, the subject's body weight, age, and sex, etc. Therefore, the dosage is not intended to limit the scope of the present invention in any aspect.

The composition of the present invention may be in the form of an injection.

The composition of the present invention may be administered to a subject by various routes. The administration may be intrathecal, intravenous, intramuscular, intraarterial, intramedullary, intradural, intraneural, intraventricular (subventricular zone), intracerebrovascular administration, and the like, and preferably, it may be directly transplanted into the dura mater of a subject in need of treatment, but is not limited thereto.

As used herein, the term "treatment" comprehensively refers to improving symptoms of a disease related to a neurodegenerative disease of the cerebellum. It may include curing (bringing the condition substantially identical to that of a normal subject) or substantially preventing (inhibiting or delaying the onset of the disease) such a disease, or alleviating the condition thereof (ameliorating or beneficially altering the condition), and may include alleviating, curing or preventing one symptom or most of symptoms resulting from a disease related to cerebellar ataxia and multiple system atrophy, but is not limited thereto.

In addition, the present invention provides a method for preparing an animal model of a neurodegenerative disease of the cerebellum, comprising injecting an inflammation-inducing substance into the cerebellum of an animal other than a human, and an animal model of the disease prepared using the same.

In one embodiment of the present invention, the inflammation-inducing substance may be LPS (lipopolysaccharide).

In one embodiment of the present invention, the animal model was prepared by directly injecting LPS into the cerebellum of a mouse using a syringe. Preferably, in one embodiment of the present invention, it was prepared by directly injecting LPS at a concentration of 5 μg/5 μl into the cerebellum of a mouse, but the concentration and injection amount of each substance and the generation may be regulated depending on the species, age, and weight of an animal model to be prepared, and the like, within the technical knowledge of one of ordinary skill in the art.

In one embodiment of the present invention, the animal model of a neurodegenerative disease induced by an inflammation-inducing substance is one in which the inflammatory response limited to the cerebellum is activated by directly administering LPS to the cerebellum, and ataxia symptoms due to dysfunction and damage of Purkinje cells observed in a patient and a animal model of cerebellar ataxia in an inflammatory environment are shown.

In one embodiment of the present invention, the activation of M1-type microglia was induced after the LPS injection, and a neuroinflammatory response of the cerebellum was induced by increasing the expression of proinflammatory cytokines such as IL-1β and TNFα or proinflammatory chemokines such as MIP-1 and MCP-1. In addition, damage or apoptosis of Purkinje cells was induced after the LPS injection.

In addition, the present invention provides a method for preparing an animal model of a neurodegenerative disease of the cerebellum, comprising directly injecting an anti-miotic agent into the cerebellum of an animal other than a human, and an animal model of the disease prepared using the same.

In one embodiment of the present invention, the anti-miotic agent may be Ara-C (cytarabine).

In one embodiment of the present invention, the animal model was prepared by directly injecting Ara-C into the abdominal cavity of a mouse using a syringe. Preferably, in one embodiment of the present invention, it was prepared by injecting 40 μg/kg of Ara-C into the abdominal cavity of an animal of 1-3 days of age, but the concentration and injection amount of each substance and the generation may be regulated depending on the species, age, and weight of an animal model to be prepared, and the like, within the technical knowledge of one of ordinary skill in the art.

In one embodiment of the present invention, the animal model of a neurodegenerative disease induced by an anti-miotic agent is one in which the development of the cerebellum is inhibited and artificial ataxia occurs by directly administering Ara-C into the abdominal cavity of an animal continuously (once a day, a total of 3 times) between day 1 and day 3 after birth, before cerebellar development progresses. In a mouse, cerebellar development occurs after 14 days of age. Purkinje cells are observed at embryo day 13, and an external granule layer, a Purkinje cell layer, and an internal granule layer of the cerebellum are formed after birth. In the case of the cerebellum, it is the only organ that develops after birth, and by directly administering an anti-miotic agent at this time, a cerebellum maturation disorder may be induced and a neurodegenerative disease may be induced.

In one embodiment of the present invention, it was confirmed anatomically that the development of the cerebellum was inhibited after the Ara-C injection, and it was found that the development of the cerebellum was inhibited through staining of the cerebellum.

In addition, the present invention provides a method for preparing an animal model of a genetic neurodegenerative disease, comprising overexpressing or inhibiting a gene of an animal other than a human, and an animal model of the disease prepared using the same.

In one embodiment of the present invention, the overexpressed gene may be SCA2.

In one embodiment of the present invention, the genetic animal model utilizes an animal model with a genetically stabilized disease through repeated backcrossing with a C57BL/6J mouse up to the fifth generation, but the concentration and injection amount of each substance and the generation may be regulated depending on the species, age, and weight of an animal model to be prepared, and the like, within the technical knowledge of one of ordinary skill in the art.

In one embodiment of the present invention, the animal model of a neurodegenerative disease caused by genetic modification is a B6D2-Tg(Pcp2-SCA2)11Plt/J mouse in which the human SCA2 gene is inserted and overexpressed into the Pcp2 promoter, and an animal model in which abnormal neurological symptoms such as ataxia and staggering appear from week 8 and about 50% of Purkinje cells are lost at week 24 to week 27 may be used.

**Effects of Invention**

The treatment method or the treatment composition using mesenchymal stem cells, according to the present invention, has remarkable effects in reducing neuroinflammation, inhibiting M1 microglia, activating M2 microglia, inhibiting apoptosis of Purkinje cells, inhibiting death of neurons, improving motor ability, and the like, and therefore may be effectively utilized in alleviating and treating neurodegenerative diseases including cerebellar ataxia and multiple system atrophy.

**Brief Description of Drawings**

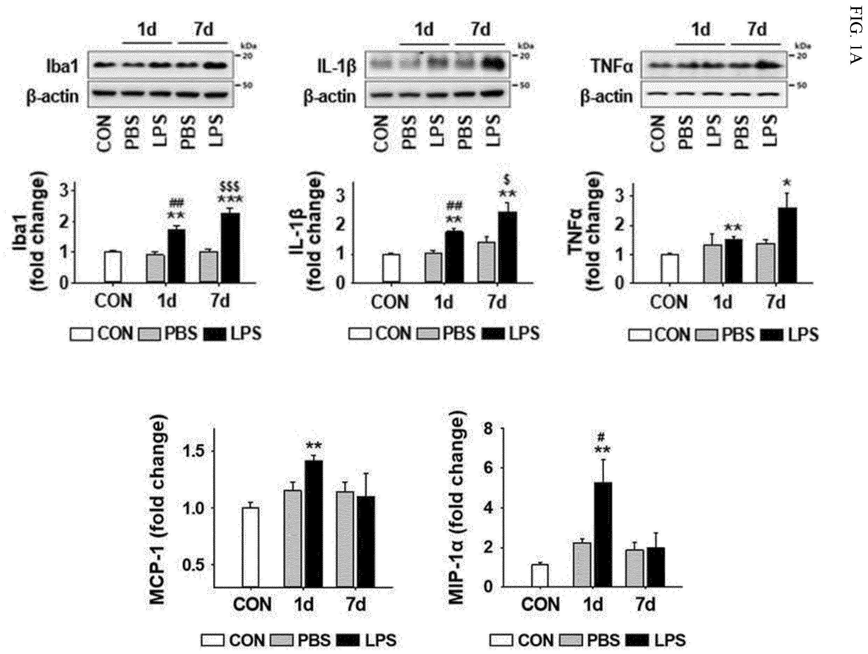

FIG. 1A shows a result obtained by confirming the construction of an animal model of a disease induced by LPS administration. It is a result obtained by confirming the expression levels of Iba1 (microglia), inflammatory cytokines (IL-1$\beta$ and TNF$\alpha$), and inflammatory chemokines (MCP-1 and MIP-1$\alpha$) in the cerebellum on day 1 and day 7 after LPS administration. Iba1: **$p < 0.01$ and ***$p < 0.001$ vs. CON, $$$p < 0.001$ vs. PBS (day 7), ##$p < 0.01$ vs. PBS (day 1). IL-1$\beta$ **$p < 0.01$ vs. CON, $p < 0.05$ vs. PBS (day 7), ##$p < 0.01$ vs. PBS (1 d). TNF$\alpha$: **$p < 0.01$ and *$p < 0.05$ vs. CON. MCP-1: **$p < 0.01$ vs. CON (t-test analysis; in each experimental group, n = 3). MIP-1$\alpha$: **$p < 0.01$ vs. CON, #$p < 0.05$ vs. PBS (day 1) (one-way ANOVA and *Tukey's post-hoc* analysis; in each experimental group, n = 3).

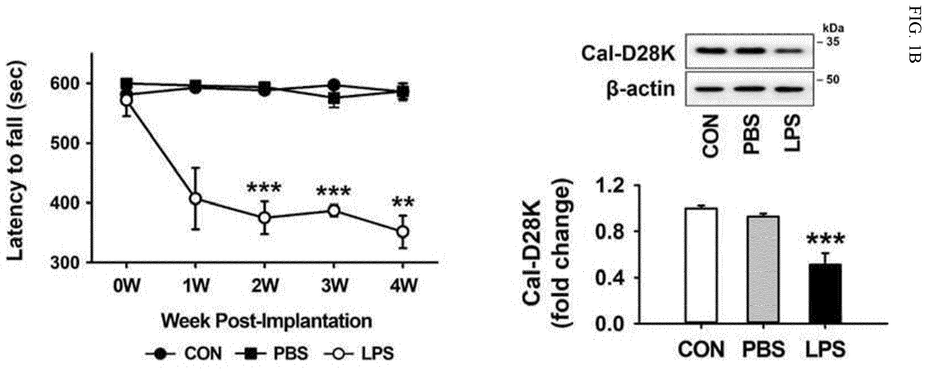

FIG. 1B shows a result obtained by confirming the construction of an animal model of a disease induced by LPS administration. It is a result obtained by confirming the expression level of Purkinje cells in the cerebellum and rotarod test for 4 weeks after LPS administration, and it is a result obtained by confirming the establishment of an animal model of a neurodegenerative disease induced by inflammation. Rotarod test for 4 weeks: **$p < 0.01$ and ***$p < 0.001$ vs. CON (t-test analysis; CON, n = 6; PBS, n = 4; LPS, n = 3). Cal-D28K: ***$p < 0.001$ vs. CON (one-way ANOVA and *Tukey's post-hoc* analysis; in each experimental group, n = 4).

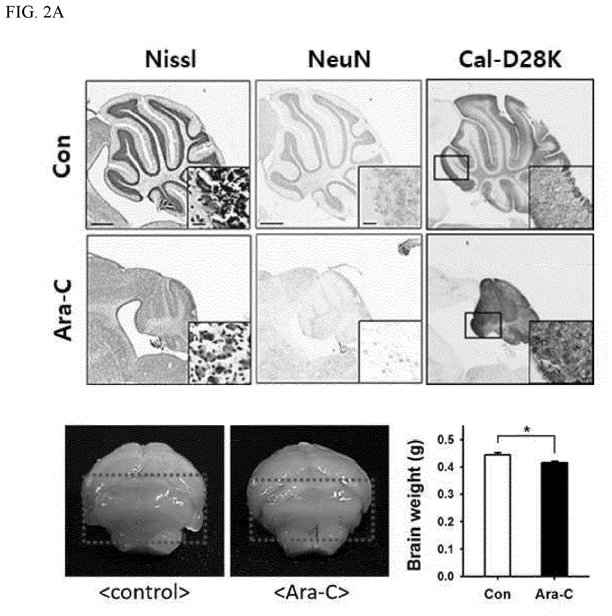

FIG. 2A shows a result obtained by confirming the construction of an animal model of a disease induced by Ara-C. It is a result obtained by confirming the adult immature cerebellum for NISSL, immunostaining, anatomical formation state, and brain weight after injection of Ara-C from day 1 to day 3 after birth. *$p < 0.05$ (t-test analysis; CON, n = 4; Ara-C, n = 4).

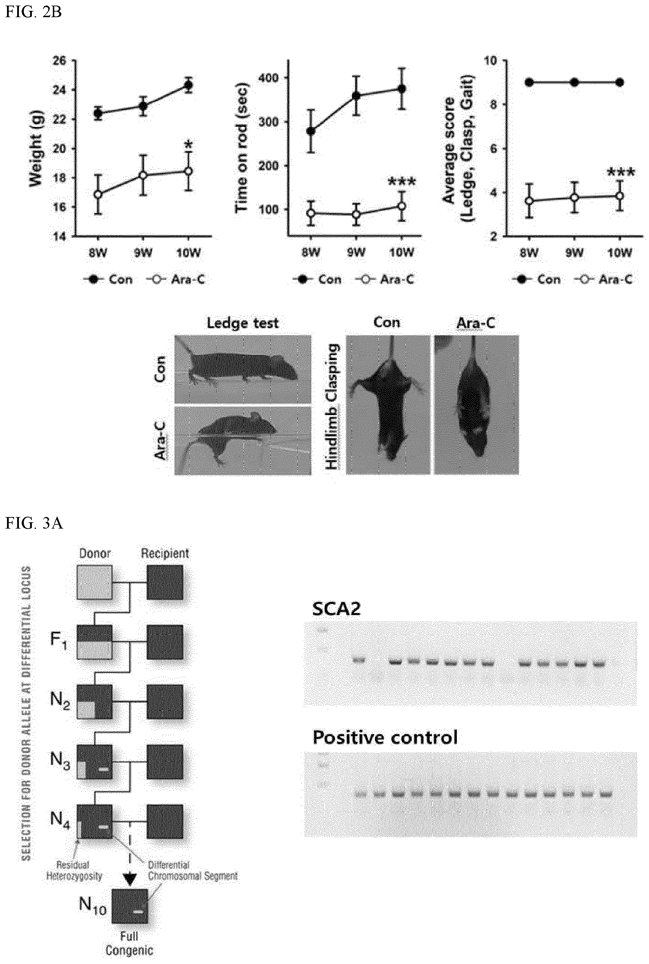

FIG. 2B shows a result obtained by confirming the construction of an animal model of a disease induced by Ara-C. It is a result obtained by evaluating the improvement of body weight and behavioral disorder of an animal of a disease induced by Ara-C, and it is a result obtained by confirming the establishment of an animal model of a neurodegenerative disease. ***$p < 0.001$ (t-test analysis; CON, n = 4; Ara-C, n = 4).

FIG. 3A shows a result obtained by establishing an animal model of a disease in which genetic stability is secured by backcrossing up to the fifth generation, which is a neurodegenerative disease SCA2 animal model through genetic modification.

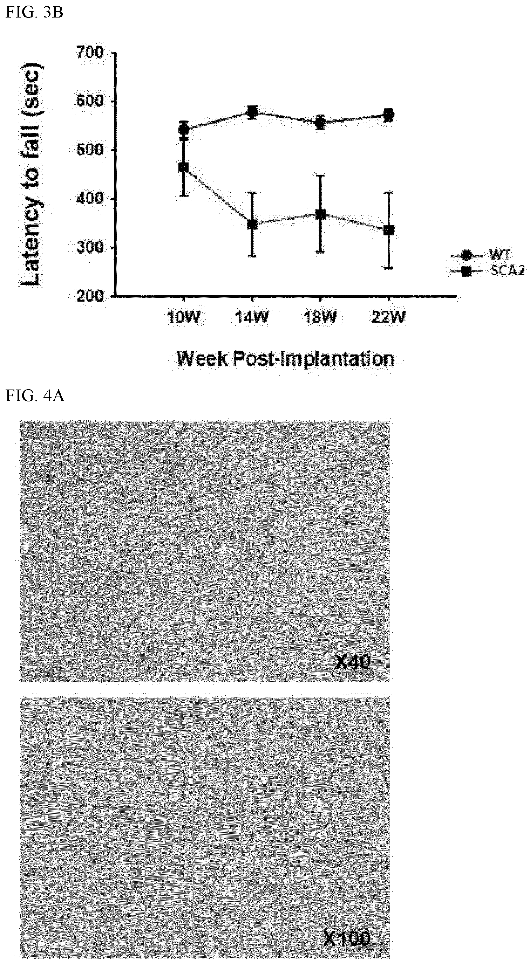

FIG. 3B shows a result obtained by performing the rotarod test to evaluate the behavioral motor of a neurodegenerative disease SCA2 animal model through genetic modification of the fifth generation.

FIG. 4A shows a result obtained by observing the spindle-shaped morphology of the initial cultured hMSCs (within P5) by microscope.

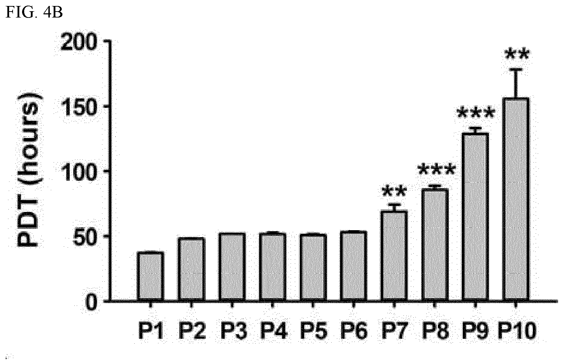

FIG. 4B shows a result obtained by analyzing the population doubling time (PDT) of hMSCs within 10 passages. **$p < 0.01$ and ***$p < 0.001$ vs. P6 (one-way ANOVA and Tukey's *post-hoc* analysis; in each experimental group, n = 4).

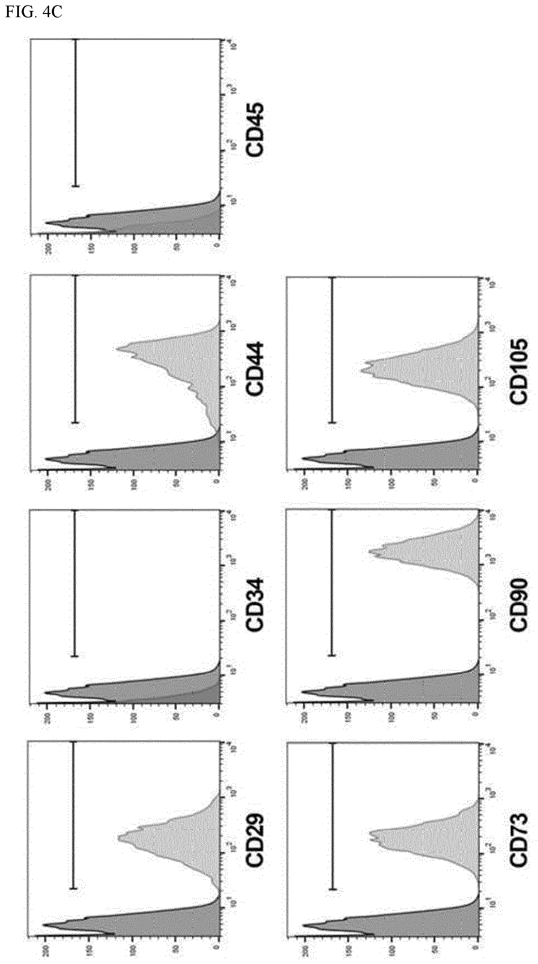

FIG. 4C shows a result obtained by analyzing the immunophenotyping for the expression profile of the cell surface markers of early stage hMSCs.

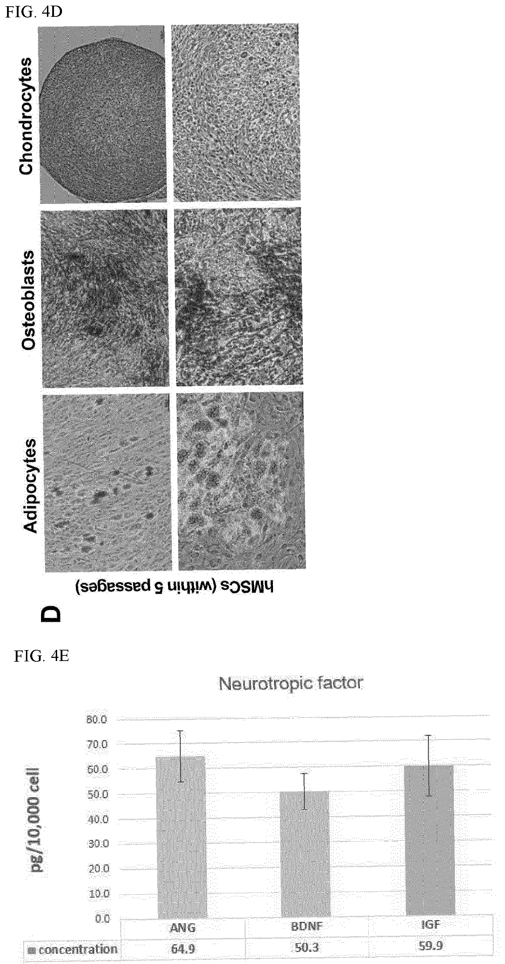

FIG. 4D shows a result obtained by confirming the multilineage differentiation ability of hMSCs.

FIG. 4E shows a result obtained by confirming the neurotropic factors expressed in hMSCs in the culture solution, and it is a result obtained by confirming the expression of ANG, BDNF, and IGF, which are well known neuroprotective and growth factors.

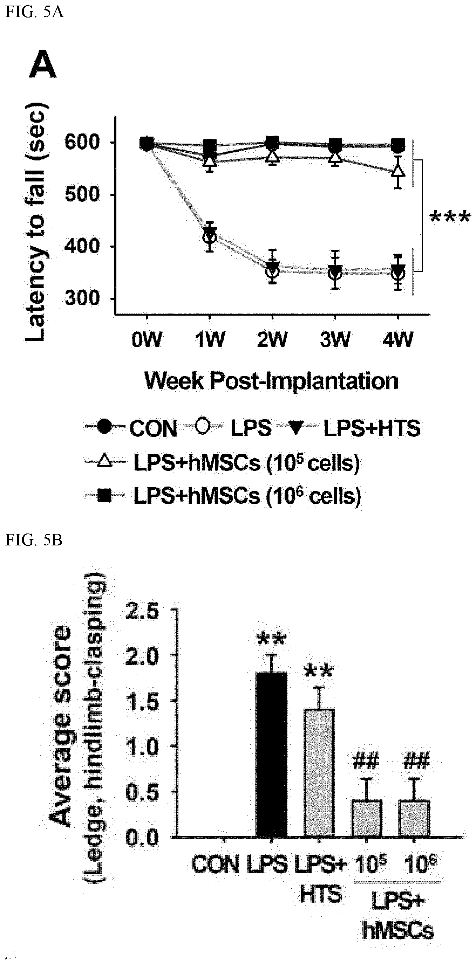

FIG. 5A shows a result obtained by performing the rotarod test for 4 weeks after transplanting hMSCs into a mouse in which the cerebellar inflammatory response is induced by LPS. Significant differences between control group (CON) or LPS+hMSCs group and LPS alone group or LPS+HTS group began to appear from 11 weeks of age throughout the experimental period ($***p < 0.001$, one-way ANOVA and *Tukey'spost-hoc* analysis; in each experimental group, n = 5).

FIG. 5B shows a result obtained by performing the evaluation of behavioral disorder improvement for 4 weeks after transplanting hMSCs. $**p < 0.01$ vs. *CON,* $^{\#\#}p < 0.01$ vs. LPS (t-test analysis; in each experimental group, n = 5).

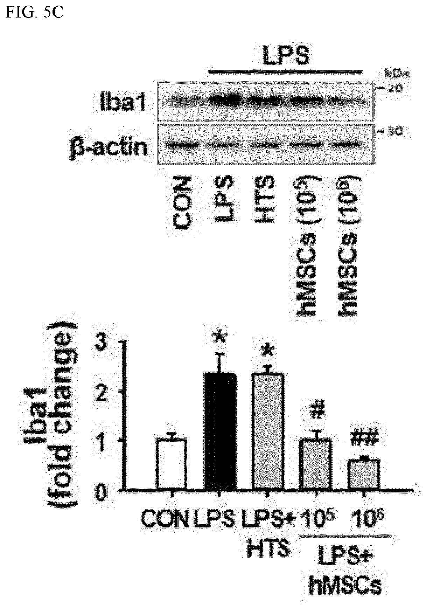

FIG. 5C shows a result obtained by confirming the expression level of Iba1 (microglia-related) in the cerebellum exposed to LPS at 7 days after transplantation of hMSCs. $*p < 0.05$ vs. CON, $^{\#}p < 0.05$ and $^{\#\#}p < 0.01$ vs. LPS (one-way ANOVA and Tukey's *post-hoc* analysis; in each experimental group, n = 3).

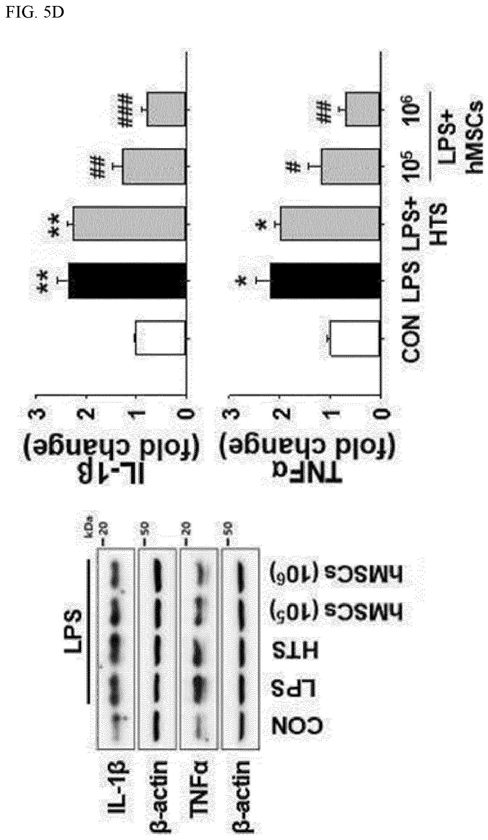

FIG. 5D shows a result obtained by confirming the expression level of pro-inflammatory cytokines (IL-1$\beta$ and TNF$\alpha$) in the cerebellum at 7 days after transplantation of hMSCs. IL-1$\beta$ $**p < 0.01$ vs. CON, $^{\#\#}p < 0.01$ and $^{\#\#\#}p < 0.001$ vs. LPS (one-way ANOVA and Tukey's *post-hoc* analysis; in each experimental group, n = 3). TNF$\alpha$: $*p < 0.05$ vs. CON, $*p < 0.05$ and $^{\#\#}p < 0.01$ vs. LPS (one-way ANOVA and Tukey's *post-hoc* analysis; in each experimental group, n = 3).

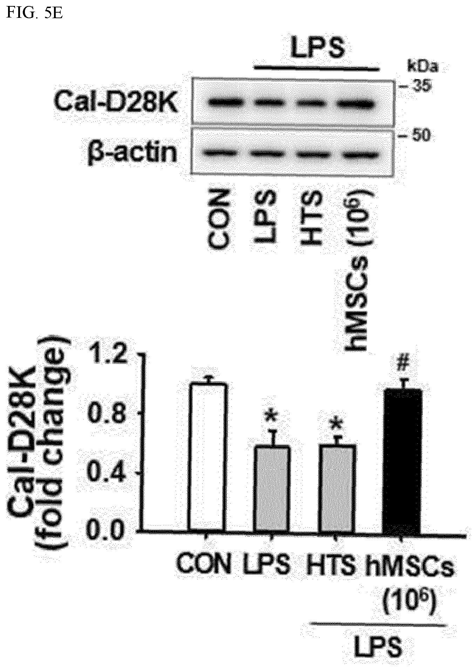

FIG. 5E shows a result obtained by confirming the expression level of Cal-D28K (Purkinje cell-related) in the cerebellum at 7 days after transplantation of hMSCs. $*p < 0.05$ vs. *CON,* $^{\#}p < 0.05$ vs. LPS (one-way ANOVA and Tukey's *post-hoc* analysis; in each experimental group, n = 4).

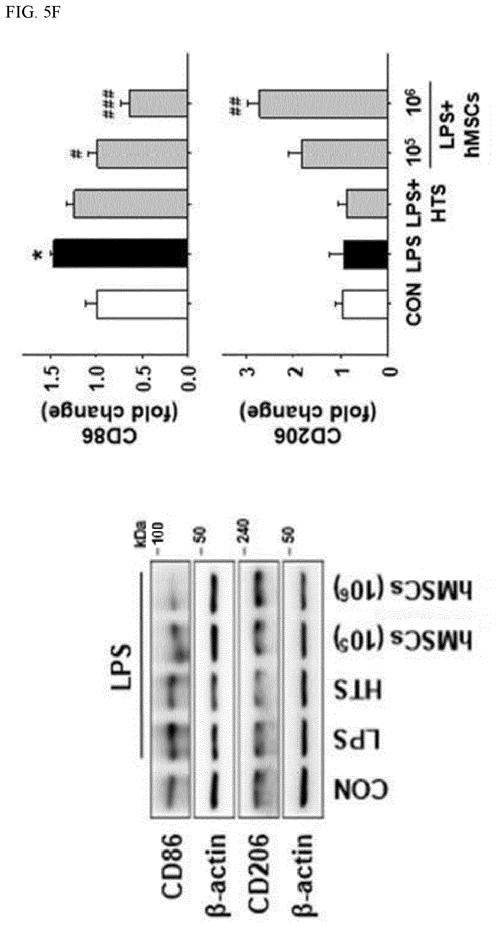

FIG. 5F shows a result obtained by confirming the expression level of CD86 and CD206 in the cerebellum at 7 days after transplantation of hMSCs. CD86: $*p < 0.05$ vs. CON, $*p < 0.05$ and $^{\#\#\#}p < 0.001$ vs. LPS (one-way ANOVA and Tukey's *post-hoc* analysis; in each experimental group, n = 3). CD206: $^{\#\#}p < 0.01$ vs. LPS (one-way ANOVA and Tukey's *post-hoc* analysis; in each experimental group, n = 3).

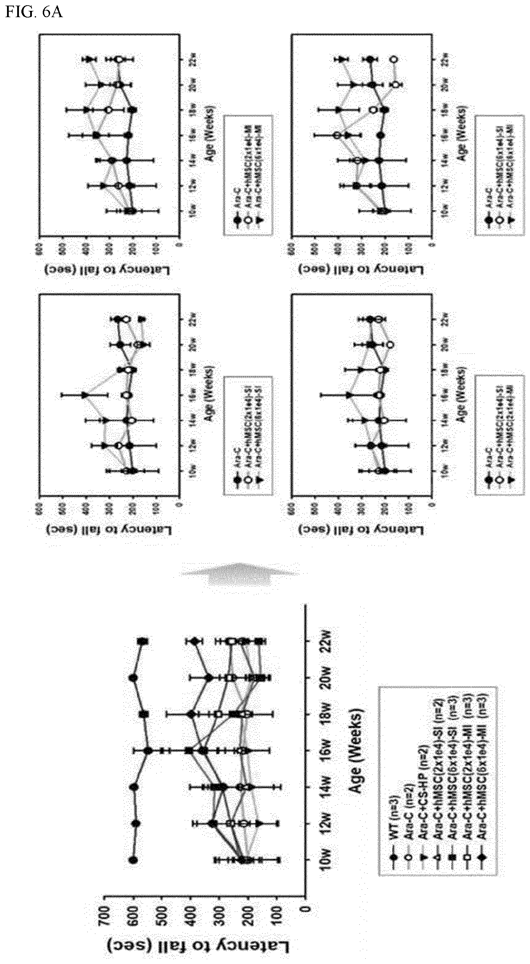

FIG. 6A shows a result obtained by confirming the effect of improving motor ability according to the concentration and frequency of administration of hMSCs in an animal model of a neurotoxicity disease induced by Ara-C (rotarod test).

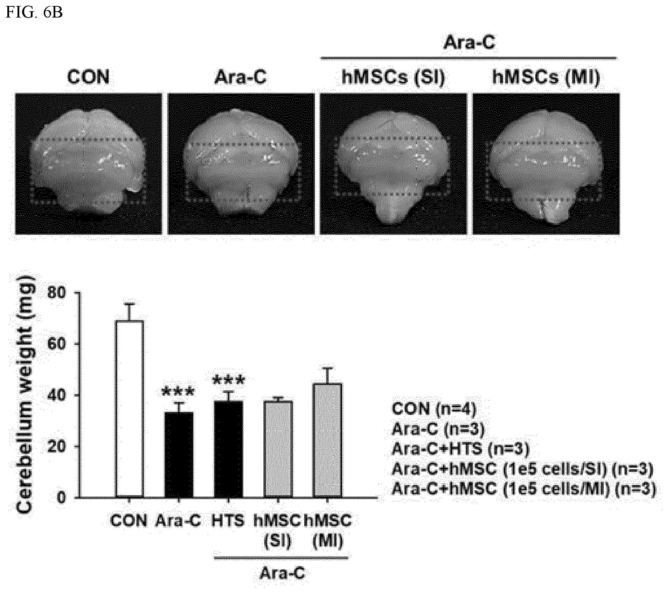

FIG. 6B shows a result obtained by confirming the effect of improving the size of the cerebellum according to the frequency of administration of hMSCs in an animal model of a neurotoxicity disease induced by Ara-C. $***p < 0.001$ vs. CON (one-way ANOVA and Tukey's *post-hoc* analysis; in each experimental group, n = 3).

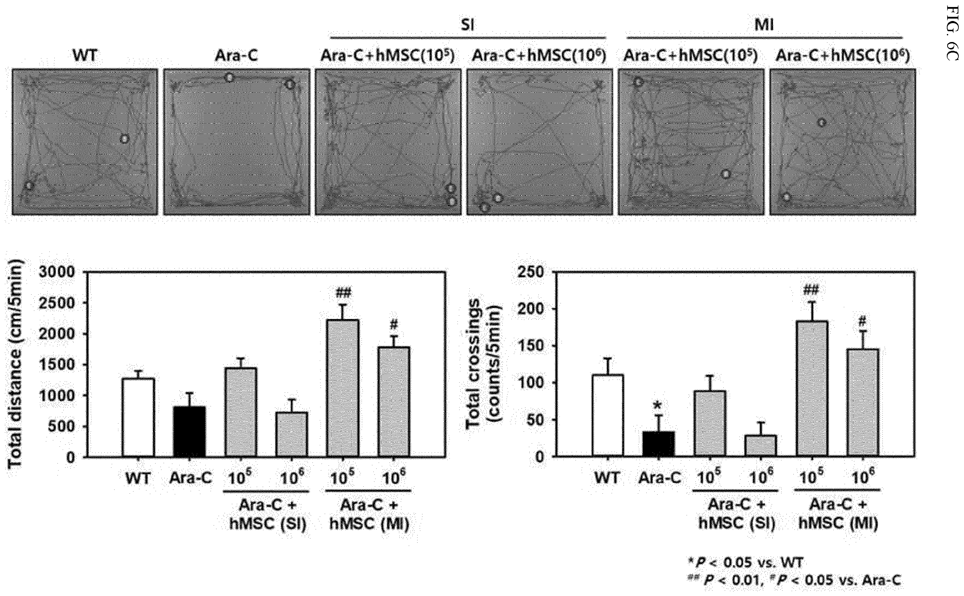

FIG. 6C shows a result obtained by comparing the effect of improving general motor activity according to the concentration and frequency of administration of hMSCs in an animal model of a neurotoxicity disease induced by Ara-C. $*p < 0.05$ vs. WT, $^{\#}p < 0.05$ and $^{\#\#}p < 0.01$ vs. Ara-C (one-way ANOVA and Tukey's *post-hoc* analysis; in each experimental group, n = 3).

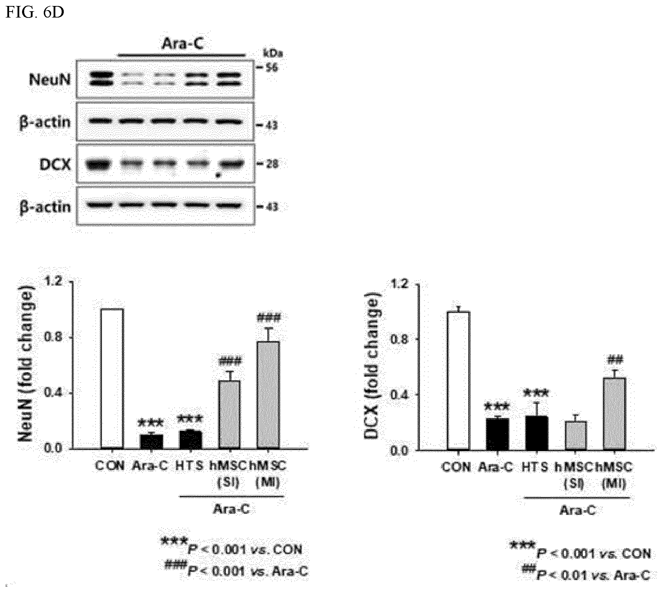

FIG. 6D shows a result obtained by confirming the increase in the amount of protein in neurons according to the frequency of administration of hMSCs in an animal model of a neurotoxicity disease induced by Ara-C. $***p < 0.001$ vs. con, $^{\#\#\#}p < 0.01$ vs. Ara-C (one-way ANOVA and Tukey's *post-hoc* analysis; in each experimental group, n = 3).

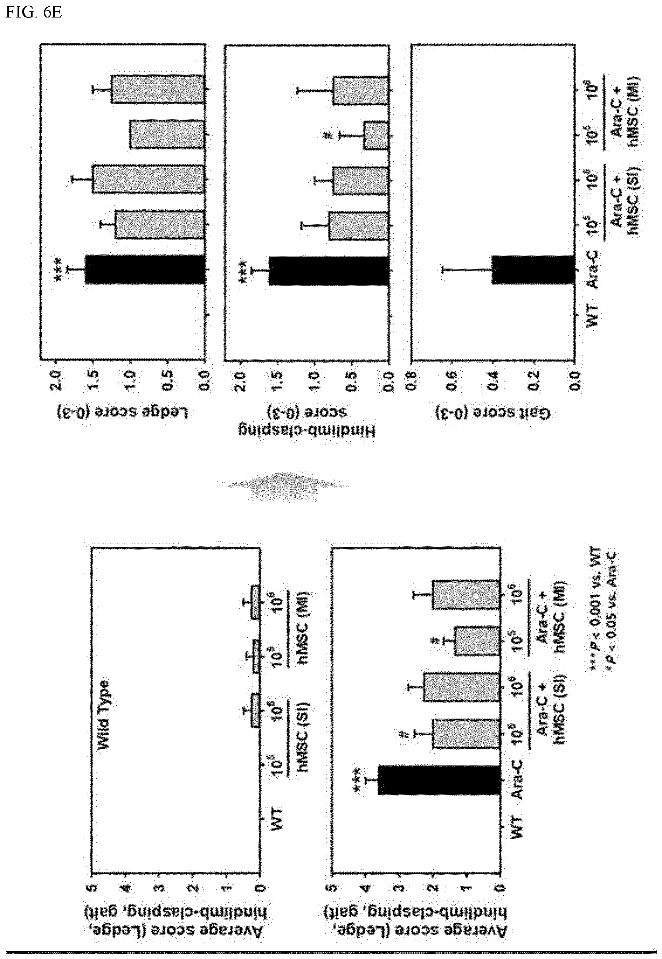

FIG. 6E shows a result obtained by confirming the improvement of neuromotor disorder according to the dose and frequency of administration of hMSCs in an animal model of a neurotoxicity disease induced by Ara-C. $***p < 0.001$ vs. WT, $^{\#}p < 0.05$ vs. Ara-C (one-way ANOVA and Tukey's *post-hoc* analysis; in each experimental group, n = 3).

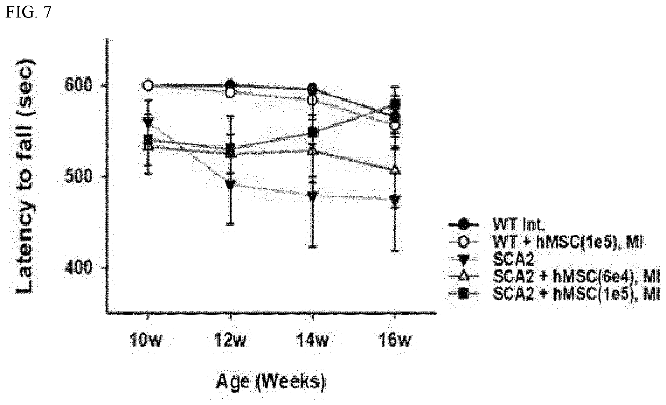

FIG. 7 shows a result obtained by confirming the effect of improving motor ability according to the dose of administration of hMSCs in an animal model of a SCA2 genetic disease caused by genetic modification.

**Best Mode for Carrying out the Invention**

**<Experimental materials and methods>**

**Animal**

Male C57BL/6 mice (Daehan Biolink, Republic of Korea) and B6D2-Tg(Pcp2-SCA2)11Plt/J mice were bred in a controlled environment with a 12-hour photoperiod, and food was distributed ad libitum. All animal experimental procedures were performed in accordance with the regulations of the Animal Experimental Ethics Committee of Kyungpook National University (No. KNU 2016-42).

**Animal model of inflammatory degenerative disease induced by LPS injection and transplantation of human mesenchymal stem cells (hMSCs)**

Inflammation-related cerebellar ataxia was induced by directly injecting lipopolyssacharide (LPS, 5 μg/5 μL) into the cerebellum of mice. Specifically, 10-week-old mice were anesthetized by injecting 115 mg/kg of ketamine (Yuhan, Republic of Korea) and 23 mg/kg of Rompun (Bayer Korea, Republic of Korea) into the abdominal cavity of 10-week-old mice, and then fixed on a stereotaxic device (David Kopf Instruments, Tujunga, CA, USA). For injection into the cerebellum, the skull was exposed through a central sagittal incision, and then burr hole opening was performed. A total of 5 μL of LPS (1 mg/mL) or phosphate buffered saline (PBS) was injected into the cerebellum through an injection pump (KD Scientific, New Hope, PA, USA) connected to a Hamilton syringe (10 μL, 30 G) (AP: - 0.25 cm; DV: -0.25 cm, relative to the lambda). The needle was left in place for 5 minutes to control back flow along the injection tube.

Next, the head of the mouse injected with LPS was turned about 90° toward the body in the stereotaxic device. The underlying dura mater was exposed, and then hMSCs ($1 \times 10^5$ cells/20 μL or $1 \times 10^6$ cells/20 μL) were transplanted into the cisterna magna using a Hamilton syringe (25 μL, 30 G) connected to an injection pump. The needle was removed after 10 minutes, and the incision site was sutured with a silk suture.

**Animal model of cerebellar ataxia induced by Ara-C injection and transplantation of human mesenchymal stem cells (hMSCs)**

Ara-C, an anti-miotic agent, was used to induce cerebellar ataxia independent of inflammation in mice. Specifically, an animal model of neurotoxicity induced cerebellar ataxia was constructed by intraperitoneal administration of Ara-C at a concentration of 40 mg/kg daily to mice from day 1 to day 3 after birth.

Next, in the same manner as above, hMSCs ($1 \times 10^5$ cells/20 μL or $1 \times 10^6$ cells/20 μL) were transplanted into the cisterna magna of the mice injected with Ara-C.

**Isolation of hMSCs and analysis of properties of stem cells**

Human bone marrow samples were secured from 8 healthy donors according to the procedure approved by the Clinical Research Review Board (IRB: 2017-11-015-007) of Kyungpook National University Chilgok Hospital.

Mononuclear cells were isolated from the bone marrow by density gradient centrifugation using Ficoll (Ficoll-Paque Premium; GE Healthcare Bio-Sciences AB), plated in CSBM-A06 medium (Corestem, Republic of Korea) at a density of about $1 \times 10^5$ cells/cm², and then cultured with 10% FBS (fetal bovine serum; Life Technologies, Grand Island, NY, USA), 2.5 mM L-alanyl-L-glutamine (Biochrom AG, Berlin, Germany), and 1% penicillin-streptomycin (Biochrom AG). Cells not attached were removed with a fresh medium, and then the medium was changed once every 3 to 4 days. Cells grown to 70-80% confluency were defined as passage 0 (passage zero, P0). Before subsequent experiments, cells were subcultured until passage 10 (P10).

**Population doubling time (PDT)**: PTD was measured in successive subculture and calculated using the following equation:

$$\frac{(T\text{-}T0) \cdot \log 2}{\log(N\text{-}N0)}$$

where T0 is the cell transplantation time, T is the cell harvest time, N0 is the initial number of cells, and N is the number of harvested cells.

**Multilineage differentiation assays**: Cell differentiation was induced according to the prior art. Specifically, hMSCs were cultured in a 24-well plate and stimulated to be differentiated according to adipogenic lineage, osteogenic lineage, and chondrogenic lineage using an hMSC functional identification kit (R&D Systems, Minneapolis, MN, USA). After differentiation, fat droplets of adipocytes were visualized using an oil red O staining reagent (Sigma, Saint Louis, MO, USA). Osteogenic differentiation was confirmed by staining calcium accumulation with alizarin red (Sigma, Saint Louis, MO, USA), and chondrogenic differentiation was confirmed by an alcian blue staining (Sigma, Saint Louis, MO, USA).

**Immunophenotyping**: In order to confirm the characteristics of hMSCs, cells within five passages were stained with the cell surface markers CD29, CD73, CD90, CD105, CD34, CD45 (BD Pharmingen, Heidelberg, Germany), and CD44 (BD Biosciences, San Diego, CA). The expression of the cell surface markers was measured using a flow cytometer (BD FACS Canto©II), and hMSCs were identified as CD29/CD44/CD73/CD 105-positive and CD34/CD45-negative cells.

**Behavior test**

For animal model behavior test, a rotarod test was performed one day before hMSC transplantation and weekly for 4 weeks after hMSC transplantation, and a simple composite phenotype scoring system was performed once every four weeks after hMSC transplantation.

**Rotarod test**: The rotarod test was used to evaluate motor coordination and balance according to the method disclosed in the prior literature (Zhang MJ, Sun JJ, Qian L, Liu Z, Zhang Z, Cao W, Li W, Xu Y: Human umbilical mesenchymal stem cells enhance the expression of neurotrophic factors and protect ataxic mice. Brain Res 2011, 1402:122-131). Experimental mice were carefully placed on a rotating rod weekly for 4 weeks. The rotation speed was increased linearly from 4 rpm to 40 rpm for 5 minutes, and the same speed (40 rpm) was maintained for 5 minutes. The time (latency) it takes for the mouse to lose balance and fall off the rod, i.e., the total time the mouse could remain on the rotating rod was recorded. In order to prevent muscle fatigue, each mouse was allowed to rest for 10 minutes between each experiment.

**Simple composite phenotype scoring system**: In order to quantitatively analyze the severity of the disease in the LPS-induced cerebellar ataxia mouse model, a conventionally known scoring system was used in combination with the ledge test and the hindlimb clasping test (Guyenet SJ, Furrer SA, Damian VM, Baughan TD, La Spada AR, Garden GA: A simple composite phenotype scoring system for evaluating mouse models of cerebellar ataxia. J Vis Exp 2010). The scoring system was done in 14-week-old mice, all tests were rated on a 0-3 point scale, and a composite phenotype score of 0-6 was added: A score of 0 means no relevant phenotype, and a score of 3 means the most severe symptom. Each test was performed three times.

**Ledge test**: Motor imbalance and ataxia were evaluated through the ledge test. A score of 0 was applied when the mouse walked along a ledge without losing overall balance, a score of 1 was applied when the mouse walked in an unbalanced position along a ledge, a score of 2 was applied when the mouse stumbled while walking along a ledge, and a score of 3 was applied when the mouse was unable to use its hindlimbs effectively.

**Hindlimb clasping test**: This test was used as a marker of disease progression in a mouse model of cerebellar ataxia (Chou AH, Yeh TH, Ouyang P, Chen YL, Chen SY, Wang HL: Polyglutamine-expanded ataxin-3 causes cerebellar dysfunction of SCA3 transgenic mice by inducing transcriptional dysregulation. Neurobiol Dis 2008, 31:89-101). A score of 0 was applied when all of the mouse's hindlimbs were consistently spread out from the abdomen, a score of 1 was applied when one hindlimb was retracted toward the abdomen for more than 50% of the measurement time, a score of 2 was applied when both hindlimbs were partially retracted toward the abdomen for more than 50% of the measurement time, and a score of 3 was applied when both hindlimbs were completely retracted toward the abdomen or touched the abdomen for more than 50% of the measurement time.

**General motility test (open field test)**: After a mouse was placed in the center of a white acrylic box (40×40×40 cm) and then allowed to move freely for 5 minutes, the movement of the mouse was measured using Smart video tracking software (Panlab Harvard Apparatus).

## Western blot analysis

For Western blotting, total cell lysates were prepared from cerebellar vermis for the mice injected with LPS and from the entire cerebellum for the mice injected with Ara-C. The cerebellar vermis was isolated, and each tissue was homogenized with a protease inhibitor cocktail (1:100, Millipore, Burlington, MA, USA) and a phosphatase inhibitor cocktail (1:100, Cell Signaling Technology) in a lysis buffer (58 mM Tris-HCl, pH 6.8; 10% glycerol; and 2% SDS). The lysate was centrifuged, and then the protein concentration was quantified using a BCA kit (Bio-Rad Laboratories, Hercules, CA, USA). Proteins were separated using gel electrophoresis and then transferred onto a membrane using an electrophoresis transfer system (Bio-Rad Laboratories). The membrane was incubated overnight at 4°C with the following primary antibodies: anti-Iba1 (anti-ionized calcium-binding adapter molecule 1, 1:1500, Wako, Osaka, Japan), anti-GFAP (anti-glial fibrillary acidic protein, 1:2000, Millipore, Billerica, MA, USA), anti-TNFa (anti-tumor necrosis factor alpha, 1:1000, Abcam, Cambridge, UK), anti-IL-1β(1 beta, 1:1000, Abcam, Cambridge, MA, USA), anti-MCP-1 (anti-monocyte chemoattractant protein 1, 1:500, Abcam, Cambridge, UK), anti-MIP-la (anti-macrophage inflammatory protein 1 alpha, 1:1000, R&D Systems, Minneapolis, MN, USA), anti-Cal-D28K (anti-calbindin-D-28K, 1:2000, Sigma, St. Louis, MO, USA), anti-cleaved caspase-3 (1:1000, Cell Signaling, Beverly, MA, USA), anti-caspase-3 (1:1000, Cell Signaling, Beverly, MA, USA), anti-CD86 (anti-cluster of differentiation 86, 1:1000, Invitrogen, Carlsbad, CA, USA), anti-CD206 (anti-cluster of differentiation 206, 1:1000, R&D Systems, Minneapolis, MN, USA), anti-iNOS (anti-inducible nitric oxide synthase, 1:1000, Abcam, Cambridge, UK), anti-IL-10 (anti-interleukin 10, 1:1000, Abcam, Cambridge, MA, USA), anti-TSG-6 (anti-TNFa stimulated gene-6, 1:1000, GeneTex, Irvine, CA, USA)), and anti-beta actin (anti-β1:2000, Santa Cruz, CA, USA).

Next, it was incubated with HRP-conjugated secondary antibody (Amersham Biosciences, Piscataway, NJ, USA), and then Western blotting was performed with a chemiluminescence Western blot detection reagent (Amersham Biosciences). For semiquantitative analysis, the band density was measured using an ImageQuant LAS 500 imager (GE Healthcare Life Science). The intensity of each protein band was calculated with Multi Gauge V3.0 software (Fuji Film, Tokyo, Japan) and normalized to the intensity of the corresponding beta actin (β band).

## Statistical analysis

All statistical analyses were performed using SigmaPlot software 12.0 (Systat Software, San Leandro, CA). Data is expressed as mean ± standard error (standard error of the mean, SEM). Statistical significance was determined using ANOVA (one-way analysis of variance), followed by Tukey's post hoc test for multiple comparisons between groups or Student's t-test for comparison between two groups.

<Experiment result>

**1. Animal model of neurodegenerative disease induced by inflammation-inducing substance (LPS)**

In order to establish an animal model of a neurodegenerative disease induced by inflammation, LPS was directly injected into the cerebellum as an effective substance for inflammatory neurodegeneration.

As shown in the Western blot results of FIG. 1A, the expression of Iba1 (microglia) was significantly increased over time by LPS injection (on day 1 after LPS-injection **$p < 0.01$ and ***$p < 0.001$, on day 7 after LPS-injection ***$p < 0.001$ vs. control group, CON). As shown in the Iba1 expression results, the expression levels of inflammatory cytokines such as IL-1β and TNFα were also significantly increased by 2.4 times and 2.6 times compared to the intact control group on day 7 after LPS injection (FIG. 1A; **$p < 0.01$, *$p < 0.05$ vs. CON). On the other hand, the expression levels of gliacytes and inflammatory cytokines in mice treated with PBS were not different from those of the control group.

In addition, in this example, the production of inflammatory chemokines such as MIP-1α and MCP-1, known as chemoattractants for inflammatory cells and mesenchymal stem cells, was confirmed in the cerebellum injected with LPS. As shown in the Western blot analysis results (FIG. 1A), the expression of MIP-1α and MCP-1 in the cerebellum was significantly increased at 1 day after LPS injection compared to the control group (**$p < 0.01$ vs. CON) and decreased therefrom after 7 days.

In order to confirm the suitability of the final animal model, the motility defect and Purkinje cell loss of the mouse were confirmed through the rotarod test. As a result, the residence time on the rotating rod was significantly reduced at 1 week after LPS injection compared to the normal control group, and the decreased value was maintained for 4 weeks (FIG. 1B; ***$p < 0.001$ 2 weeks and 3 weeks after LPS injection vs. CON, **$p < 0.01$ 4 weeks after LPS injection vs. CON). In addition, calbindin protein was significantly reduced in the cerebellum on day 7 after LPS injection in the mice injected with LPS compared to the normal control group, indicating a loss of Purkinje cells (FIG. 1B; ***$p < 0.001$ vs. CON).

**2. Animal model of neurodegenerative disease induced by anti-miotic agent**

In order to establish an animal model of a neurodegenerative disease induced by an anti-miotic agent, Ara-C was intraperitoneally injected daily for 1 to 3 days after birth.

As shown in the results of FIG. 2A, the structure of the cerebellum, neurons, and Purkinje cells were confirmed through histopathological staining. As a result, it was confirmed that the development of the cerebellum of the animals administered with Ara-C was inhibited, and it was confirmed that neurons and Purkinje cells of the cerebellum were not formed.

Additionally, the disease animal model was evaluated through behavioral evaluation. As a result, it was confirmed that there was a behavioral imbalance compared to the control group, such as parallel, hanging, and gait (FIG. 2B).

**3. Animal model of genetic neurodegenerative disease through genetic modification**

In order to establish an animal model of a genetic neurodegenerative disease through genetic modification, B6D2-Tg(Pcp2-SCA2)11Plt/J mouse was established by inserting and overexpressing the human SCA2 gene into the mouse Pcp2 promoter. However, the secured B6D2-Tg(Pcp2-SCA2)11Plt/J mouse was an animal model established by fertilization of C57BL/6J ovum and DBA/2J sperm. Because the genetic background was not uniform, crossing was carried out until the fifth generation, which showed 94% genetic stability through repeated backcrossing with C57BL/6J mice.

Therefore, as shown in FIG. 3A, genetic stabilization was performed through backcrossing, and then the SCA2 gene expression was confirmed to establish an animal model of a genetic neurodegenerative disease.

Additionally, as shown in the results of FIG. 3B, it was confirmed that there was a behavioral imbalance compared to the control group (Wild type, WT) animals through the rotarod test, which is a behavioral evaluation.

**4. Properties of mesenchymal stem cell therapeutic agent (hMSC)**

In order to confirm the properties of the mesenchymal stem cell therapeutic agent isolated from the bone marrow, the properties of the stem cell therapeutic agent were confirmed through analysis of shape, marker, and differentiation ability, and the functional properties of the stem cells were also confirmed through the secreted cytokines.

First, the characteristics of hMSCs were determined by the morphology of fibroblastoid, the expression pattern of hMSC-related surface markers, and differentiation possibility according to the criteria of the International Society for Cellular Therapy (ISCT).

In hMSCs isolated from the bone marrow, the original shape of spindle-shaped fibroblast-shaped stem cells could be visually confirmed (FIG. 4A). In addition, the PDT of hMSCs for each passage was evaluated to confirm the proliferative capacity of stem cells. Although hMSCs at the early passage stage showed high proliferative capacity (37 - 51 hours), it was confirmed that the proliferative capacity of hMSCs was poor in the late passage stage (after Passage 7) (FIG. 4B).

As a result of flow cytometry, 95% or more of the early hMSCs were positive for hMSC-related CD markers such as CD29, CD44, CD73, CD90, and CD105, whereas they were negative for hematopoietic stem cell-related CD markers such as CD34 and CD45 (FIG. 4C).

In order to confirm the differentiation ability of bone marrow-derived hMSCs, stem cells at the early stage (Passage 2 to 4) were cultured in adipogenic medium, osteogenic medium and chondrogenic medium for 2 to 4 weeks to confirm differentiation into adipocytes, osteoblasts and chondrocytes. Through this, it was found that the mesenchymal stem cell therapeutic agent isolated/produced with the unique properties of mesenchymal stem cells well maintained the properties of stem cells until the 7th passage (FIG. 4D).

In order to predict the therapeutic effect in a neurodegenerative disease, the neurotropic factor was identified to identify the secreted protein of the mesenchymal stem cell therapeutic agent. As a result, the expression of ANG, BDNF, and IGF, which are closely related to neuroprotective factors and growth and differentiation of neurons, was confirmed. It is thought that the mesenchymal stem cell therapeutic agent may be beneficial for a neurodegenerative disease (FIG. 4E).

Based on the above results, only hMSCs within passage 5 (PI to P5) were used in subsequent experiments.

### 5. Therapeutic effect of hMSCs in animal model of neurodegenerative disease induced by inflammation

In order to evaluate whether intrathecal transplanted hMSCs have beneficial effects in an animal model of a neurodegenerative disease induced by LPS, motor coordination and balance were evaluated using the rotarod test weekly for 4 weeks after hMSC transplantation using 10-week-old mice. The control group mice were administered with HTS (HypoThermosol), an optimal preservative used to store stem cells at low temperature. As a result, before LPS administration, mice in all groups showed a similar level of motor coordination, but after LPS administration, it was confirmed that the disease animal had gradually decreased motility compared to the control group. When the stem cell therapeutic agent was transplanted at a low dose ($1\times10^5$ cells/mouse) and at a high dose ($1\times10^6$ cells/mouse), it was confirmed that the motor performance capability of the disease animal was significantly enhanced compared to the control group (FIG. 5A). When behavioral disorders were evaluated through the composite ataxia phenotype scoring system, it was confirmed that a behavioral disorder was significantly improved in a group administered with the mesenchymal stem cell therapeutic agent (FIG. 5B).

Next, by confirming not only behavioral indicators but also the reduction of inflammatory response, inhibition of apoptosis of Purkinje cells, and the expression level of M1- and M2-type microglia, the effect of a mesenchymal stem cell therapeutic agent in various mechanisms was confirmed.

In order to confirm the reduction of the inflammatory response of the mesenchymal stem cell therapeutic agent, the activation of gliacytes induced by the LPS inflammatory response was confirmed. It was confirmed that Iba1 was significantly reduced in a group administered with the mesenchymal stem cell therapeutic agent compared to the control group (FIG. 5C), and it was also confirmed that the inflammatory cytokines IL-1β and TNF-α were also reduced in the cerebellum (FIG. 5D). This is confirmed by anti-inflammatory and immune-modulating effects of the mesenchymal stem cell therapeutic agent.

In addition, the protective effect of a stem cell therapeutic agent capable of inhibiting and protecting the apoptosis of Purkinje cells induced by inflammatory response was confirmed. The Purkinje cell marker Cal-D28K was reduced in the cerebellum of the disease animal, but it was confirmed that the expression of the marker was higher in a group administered with the mesenchymal stem cell therapeutic agent compared to the control group (FIG. 5E). In addition, in order to confirm the change in polarization of M1- and M2-type microglia, when CD86 as an M1-type marker and CD206 as an M2-type marker were identified, it was confirmed that M1-type microglia of CD86 were reduced and M2-type microglia of CD206 were increased (FIG. 5F). This indicates that M1-type microglia are polarized into M2-type microglia related to tissue regeneration, and this can represent the secondary therapeutic improvement effect of stem cells.

### 6. Therapeutic effect of hMSCs in animal model of neurodegenerative disease induced by anti-miotic agent

In order to compare the effect of improving motor ability according to the administration method of a stem cell therapeutic agent at a low dose ($2\times10^4$ or $6\times10^4$ cells) in an animal model of cerebellar ataxia induced by Ara-C, an anti-miotic agent, the rotarod test was performed every 2 weeks for 12 weeks from the time of stem cell administration (10 weeks of age), and motor coordination and balance were evaluated. In this example, stem cells were administered in the following four ways:

① once administration of $2\times10^4$ cells (n=2)
② 3 times repeated administration of $2\times10^4$ cells at a 4-week interval (10w, 14w, 18w) (n=3)
③ once administration of $6\times10^4$ cells (n=3)
④ 3 times repeated administration of $6\times10^4$ cells at a 4-week interval (10w, 14w, 18w) (n=3)

As a result, the cerebellar ataxia model (Ara-C) mice administered with Ara-C failed to maintain balance and fell off in the rotarod test compared to the normal control group (WT). On the other hand, when the Ara-C model mice were administered with hMSCs, there was a difference in the enhancement of motor performance capability according to the administration dose and method. Specifically, when the mice were administered with $2\times10^4$ cells, there was no significant difference compared to the mice which were not administered with the stem cells. When $2\times10^4$ cells were repeatedly administered and when $6\times10^4$ cells were administered once, the balance ability was gradually increased until 16 weeks of age, but after that, it was reduced again. Finally, it was confirmed that the balance ability was continuously increased when $6\times10^4$ cells were repeatedly administered (FIG. 6A). Through this, it was confirmed that, in relation to the effect of mesenchymal stem cells in the disease animal, the behavioral improvement effect appeared when at least $6\times10^4$ cells were administered once or repeatedly.

In addition, in order to confirm anatomical changes, changes in the size and weight of the cerebellum after administration of the mesenchymal stem cell therapeutic agent were confirmed. As a result, it was confirmed that the size of the cerebellum was significantly reduced in the cerebellar ataxia model (Ara-C) mice administered with Ara-C compared to the normal control group (WT), but the size of the cerebellum was slightly increased after administration of stem cells (FIG. 6B).

In addition, in order to measure general motor activity, the open field test was performed. In this example, stem cells were administered once or repeatedly at two concentrations ($1\times10^5$ or $1\times10^6$ cells), and the results were compared. Specifically, stem cells at each concentration were administered once at 10 weeks of age or administered repeatedly 3 times at a 4-week interval, and then at 22 weeks of age, general motility was measured through the open field test. As a result, it was confirmed that the motility was significantly reduced in the cerebellar ataxia model (Ara-C) mice administered with Ara-C compared to the normal control group (WT), but the motility was enhanced after administration of stem cells. The effect of improving motor activity was more pronounced when the stem cells were administered repeatedly than when the stem cells were administered once, and in both the single administration and the repeated administration, when the stem cells were administered at a concentration of $1\times10^5$ cells, the effect of improving motor activity was higher (FIG. 6C).

The effect of the therapeutic agent was also confirmed by differences in the protein. After administration of mesenchymal stem cells at the concentration of $1\times10^5$ cells in the disease animal, it was confirmed that the expression of the neuron protein NeuN and the neuroblast and neuron progenitor cell marker DCX (doublecortin) was significantly increased. This is considered to be a therapeutic effect due to improvement of maturation disorder of the cerebellum (FIG. 6D).

In the composite phenotype scoring system, which is a neurobehavioral test, the mesenchymal stem cell therapeutic agent was administered once or repeatedly at two concentrations ($1\times10^5$ or $1\times10^6$ cells), and the results were compared. Specifically, stem cells at each concentration were administered once at 10 weeks of age or administered repeatedly 3 times at a 4-week interval, and then at 22 weeks of age, the effect of improving behavioral disorders was measured through the composite phenotype scoring system. As a result, it was confirmed that the total mean score was significantly reduced after administration of stem cells compared to the cerebellar ataxia model (Ara-C) mice administered with Ara-C, and neurobehavioral symptoms were more alleviated when administered repeatedly than when administered once, and when administered at a concentration of $1\times10^5$ cells than when administered at a concentration of $1\times10^6$ cells (FIG. 6E).

## 7. Therapeutic effect of hMSCs in animal model of SCA2 neurodegenerative disease through genetic modification

In order to evaluate whether intrathecal transplanted hMSCs have therapeutic effects in an animal model of a genetic modification disease (SCA2), motor coordination and balance were evaluated using the rotarod test weekly for 4 weeks after transplantation of hMSCs into 10-week-old mice. As a result, behavioral imbalance was confirmed in the SCA2 gene mutation animal model. In addition, when the mesenchymal stem cell therapeutic agent was transplanted at a low dose ($6\times10^4$ cells/mouse) and at a high dose ($1\times10^5$ cells/mouse), it was confirmed that the motor performance capability of the disease animal was enhanced compared to the control group. This is the result of confirming the therapeutic effect of mesenchymal stem cells in the animal model of genetic diseases as well as in the animal model of diseases artificially induced by substances, and it was confirmed that the mesenchymal stem cell therapeutic agent has a therapeutic effect on various pathological mechanisms (FIG. 7).

### Industrial applicability

The treatment method or the treatment composition using mesenchymal stem cells, according to the present invention, has remarkable effects in reducing neuroinflammation, inhibiting M1 microglia, activating M2 microglia, inhibiting apoptosis of Purkinje cells, inhibiting death of neurons, improving motor ability, and the like, and therefore may be effectively utilized in alleviating and treating neurodegenerative diseases including cerebellar ataxia and multiple system atrophy, and thus has high industrial applicability.

### Claims

1. A method for treating neurodegenerative diseases of the cerebellum, comprising:
   administering a composition comprising stem cells as an active ingredient to a patient having a neurodegenerative disease of the cerebellum.

2. The method for treating neurodegenerative diseases of the cerebellum according to claim 1, wherein the stem cell is an adult stem cell.

3. The method for treating neurodegenerative diseases of the cerebellum according to claim 2, wherein the adult stem cell is at least one selected from the group consisting of a mesenchymal stem cell (MSC), a mesenchymal stromal cell, and a multipotent stem cell.

4. The method for treating neurodegenerative diseases of the cerebellum according to claim 1, wherein the stem cells induce anti-inflammatory activity of the cerebellum.

5. The method for treating neurodegenerative diseases of the cerebellum according to claim 1, wherein the stem cells reduce the expression of inflammatory cytokines or inflammatory chemokines.

6. The method for treating neurodegenerative diseases of the cerebellum according to claim 1, wherein the stem cells inhibit the activation of inflammatory neuroglia and activate anti-inflammatory neuroglia.

7. The method for treating neurodegenerative diseases of the cerebellum according to claim 1, wherein the stem cells inhibit damage of a Purkinje cell.

8. The method for treating neurodegenerative diseases of the cerebellum according to claim 1, wherein the neurodegenerative disease of the cerebellum is at least one selected from the group consisting of a neurodegenerative disease induced by inflammation, a neurodegenerative disease induced by toxins, and a neurodegenerative disease caused by genetic modification.

9. The method for treating neurodegenerative diseases of the cerebellum according to claim 1, wherein the neurodegenerative disease of the cerebellum includes cerebellar ataxia or multiple system atrophy.

10. The method for treating neurodegenerative diseases of the cerebellum according to claim 1, wherein 90% or more of the stem cells express a positive surface marker including CD29, CD44, CD73, CD90 or CD105, or 5% or less of the stem cells express a negative surface marker including CD34 or CD45.

11. The method for treating neurodegenerative diseases of the cerebellum according to claim 1, wherein the administration is performed via at least one route of administration selected from the group consisting of intrathecal, intravenous, intramuscular, intraarterial, intramedullary, intradural, intraneural, intraventricular and intracerebrovascular.

12. A composition for alleviating or treating symptoms of neurodegenerative diseases of a cerebellum, comprising stem cells as an active ingredient.

13. The composition according to claim 12, wherein the stem cell is an adult stem cell.

14. The composition according to claim 12, wherein the adult stem cell is at least one selected from the group consisting of a mesenchymal stem cell (MSC), a mesenchymal stromal cell, and a multipotent stem cell.

15. The composition according to claim 12, wherein the stem cells induce anti-inflammatory activity of the cerebellum.

16. The composition according to claim 12, wherein the stem cells reduce the expression of inflammatory cytokines or inflammatory chemokines.

17. The composition according to claim 12, wherein the stem cells inhibit the activation of inflammatory neuroglia and activate anti-inflammatory neuroglia.

18. The composition according to claim 12, wherein the stem cells inhibit damage of Purkinje cell.

19. The composition according to claim 12, wherein the neurodegenerative disease of the cerebellum is at least one selected from the group consisting of a neurodegenerative disease induced by inflammation, a neurodegenerative disease induced by toxins, and a neurodegenerative disease caused by genetic modification.

20. The composition according to claim 12, wherein 90% or more of the stem cells express a positive surface marker including CD29, CD44, CD73, CD90 or CD105, or 5% or less of the stem cells express a negative surface marker including CD34 or CD45.

21. The composition according to claim 12, wherein the neurodegenerative disease of the cerebellum includes cerebellar ataxia or multiple system atrophy.

FIG. 1A

FIG. 1B

FIG. 2A

FIG. 2B

FIG. 3A

FIG. 3B

FIG. 4A

FIG. 4B

FIG. 4C

FIG. 4D

hMSCs (within 5 passages)

D

FIG. 4E

Neurotropic factor

| | ANG | BDNF | IGF |
|---|---|---|---|
| concentration | 64.9 | 50.3 | 59.9 |

FIG. 5A

FIG. 5B

FIG. 5C

FIG. 5D

FIG. 5E

FIG. 5F

FIG. 6A

FIG. 6B

FIG. 6C

FIG. 6D

FIG. 6E

FIG. 7

<div align="center">

**INTERNATIONAL SEARCH REPORT**

</div>

| International application No. |
|---|
| **PCT/KR2021/000263** |

**A. CLASSIFICATION OF SUBJECT MATTER**

**A61K 35/28**(2006.01)i; **A61K 9/00**(2006.01)i; **A61P 25/28**(2006.01)i

According to International Patent Classification (IPC) or to both national classification and IPC

**B. FIELDS SEARCHED**

Minimum documentation searched (classification system followed by classification symbols)

A61K 35/28(2006.01); A61K 35/12(2006.01); A61K 35/54(2006.01); C12N 5/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 소뇌(cerebellum), 소뇌실조증(cerebellar ataxia, CA), 다계통위축증(multiple system atrophy), 중간엽 줄기세포(mesenchymal stem cell, MSC), 지질다당질(lipopolysaccharide, LPS)

**C. DOCUMENTS CONSIDERED TO BE RELEVANT**

| Category* | Citation of document, with indication, where appropriate, of the relevant passages | Relevant to claim No. |
|---|---|---|
| X | YOON, Dongyeong et al. Beneficial effects of hMSC treatment in LPS-induced animal model of cerebellar ataxia. IBRO Reports P29.11. September 2019, vol. 6, page S424.<br>See entire document. | 12-21 |
| X | US 8637003 B2 (LEE, Phil Hyu et al.) 28 January 2014 (2014-01-28)<br>See abstract; and claims 1-7. | 12-14,20,21 |
| X | KR 10-2017-0093737 A (KYUNGPOOK NATIONAL UNIVERSITY INDUSTRY-ACADEMIC COOPERATION FOUNDATION) 16 August 2017 (2017-08-16)<br>See abstract; and claims 1-9. | 12,13,21 |
| A | KR 10-2014-0104377 A (SAMSUNG LIFE PUBLIC WELFARE FOUNDATION) 28 August 2014 (2014-08-28)<br>See abstract; and claims 1-7. | 12-21 |
| A | NAKAMURA, Kazuhiro et al. Mesenchymal stem cell as a potential therapeutic tool for spinocerebellar ataxia. Cerebellum. 04 October 2014(online publication date), vol. 14, pp. 165-170.<br>See abstract; and pages 165-170. | 12-21 |

☑ Further documents are listed in the continuation of Box C.     ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **27 April 2021** | **27 April 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2021/000263** |

**C.    DOCUMENTS CONSIDERED TO BE RELEVANT**

| Category* | Citation of document, with indication, where appropriate, of the relevant passages | Relevant to claim No. |
|---|---|---|
| PX | NAM, Youngpyo et al. Therapeutic effects of human mesenchymal stem cells in a mouse model of cerebellar ataxia with neuroinflammation. Journal of Clinical Medicine. 13 November 2020, vol. 9, no. 11 , article no. 3654, inner pp. 1-18.<br>    See abstract; and inner pages 1-18. | 12-21 |
| PX | KIM, Jong-Heon et al. Characterization of mesenchymal stem cells derived from patients with cerebellar ataxia: downregulation of the anti-inflammatory secretome profile. Cells. 15 January 2020, vol. 9, no. 1, article no. 212, inner pp. 1-16.<br>    See abstract; and inner pages 1-16. | 12-21 |

Form PCT/ISA/210 (second sheet) (July 2019)

## INTERNATIONAL SEARCH REPORT

International application No.

**PCT/KR2021/000263**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **1-11**
   because they relate to subject matter not required to be searched by this Authority, namely:

   Claims 1-11 pertain to a method for treatment of the human body by surgery or therapy, as well as a diagnostic method (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2019)

**INTERNATIONAL SEARCH REPORT**
Information on patent family members

International application No.

**PCT/KR2021/000263**

| Patent document cited in search report | | | Publication date (day/month/year) | Patent family member(s) | | | Publication date (day/month/year) |
|---|---|---|---|---|---|---|---|
| US | 8637003 | B2 | 28 January 2014 | US | 2009-269311 | A1 | 29 October 2009 |
| KR | 10-2017-0093737 | A | 16 August 2017 | EP | 3412296 | A1 | 12 December 2018 |
| | | | | EP | 3412296 | A4 | 11 September 2019 |
| | | | | KR | 10-1865404 | B1 | 07 June 2018 |
| | | | | US | 2019-0030082 | A1 | 31 January 2019 |
| | | | | WO | 2017-135753 | A1 | 10 August 2017 |
| KR | 10-2014-0104377 | A | 28 August 2014 | EP | 2959909 | A1 | 30 December 2015 |
| | | | | EP | 2959909 | A4 | 17 August 2016 |
| | | | | EP | 2959909 | B1 | 18 December 2019 |
| | | | | KR | 10-1654970 | B1 | 06 September 2016 |
| | | | | US | 10493104 | B2 | 03 December 2019 |
| | | | | US | 2016-0000831 | A1 | 07 January 2016 |
| | | | | WO | 2014-129792 | A1 | 28 August 2014 |

Form PCT/ISA/210 (patent family annex) (July 2019)

# REFERENCES CITED IN THE DESCRIPTION

**Patent documents cited in the description**

- KR 1020200002758

**Non-patent literature cited in the description**

- **ZHANG MJ ; SUN JJ ; QIAN L ; LIU Z ; ZHANG Z ; CAO W ; LI W ; XU Y.** Human umbilical mesenchymal stem cells enhance the expression of neurotrophic factors and protect ataxic mice. *Brain Res,* 2011, vol. 1402, 122-131
- **GUYENET SJ ; FURRER SA ; DAMIAN VM ; BAUGHAN TD ; LA SPADA AR ; GARDEN GA.** A simple composite phenotype scoring system for evaluating mouse models of cerebellar ataxia. *J Vis Exp,* 2010
- **CHOU AH ; YEH TH ; OUYANG P ; CHEN YL ; CHEN SY ; WANG HL.** Polyglutamine-expanded ataxin-3 causes cerebellar dysfunction of SCA3 transgenic mice by inducing transcriptional dysregulation. *Neurobiol Dis,* 2008, vol. 31, 89-101